Europäisches Patentamt

European Patent Office

Office européen des brevets

(19)

(11) Numéro de publication: **0 461 330 A1**

## (12) DEMANDE DE BREVET EUROPEEN

(21) Numéro de dépôt: **90810421.9**

(22) Date de dépôt: **11.06.90**

(51) Int. Cl.⁵: **C07K 15/06**, A61K 7/00

(43) Date de publication de la demande:
**18.12.91 Bulletin 91/51**

(84) Etats contractants désignés:
**AT BE CH DE DK ES FR GB GR IT LI LU NL SE**

(71) Demandeur: **SEROLAB SA**
**Avenue C.-F. Ramuz 43**
**CH-1009 Pully(CH)**

Demandeur: **Appelboom, Thierry, Prof. Dr.**
**27, Avenue Erasme**
**B-1810 Wemmel(BE)**

(72) Inventeur: **Appelboom, Thierry**
**Avenue Erasme 27**
**B-1810 Wemmel(BE)**

(74) Mandataire: **AMMANN PATENTANWAELTE AG**
**BERN et al**
**Schwarztorstrasse 31**
**CH-3001 Bern(CH)**

(54) **Fraction inhibitrice de protéases.**

(57) On décrit et revendique une fraction inhibitrice de protéases pouvant être préparée à partir d'un sérum sanguin et contenant des facteurs exerçant une action inhibitrice de l'élastase au moins. Le sérum chevalin contient un facteur supplémentaire inconnu jusqu'ici. Le procédé comprend l'enlèvement sélectif de l'albumine, la concentration isoélectrique des inhibiteurs, et leur isolation à la fin du procédé. Des préparations cosmétiques sont décrites contenant ledit mélange d'inhibiteurs ou le nouveau facteur "horse inhibitor" seul ou en mélange.

EP 0 461 330 A1

La présente invention appartient au domaine de la biochimie. Elle concerne une composition inhibitrice de protéases et son utilisation en cosmétique.

Les inhibiteurs de protéases qui sont en somme des anti-enzymes, sont déjà connus en soi. Le maintien de la santé humaine et animale requiert une balance entre les systèmes offensifs et défensifs dans le corps d'un être vivant. Une telle balance récemment découverte est celle entre les enzymes qui sont les biocatalyseurs de la scission des protéines, donc les protéases, et les antiprotéases qui inhibent l'action de ces enzymes. Des détails se rapportant à ces balances biomoléculaires ont été décrits par R.W. Carrel dans J. Clin. Invest. 78, 1427-31 (1986).

Un représentant important des inhibiteurs des protéases est l'alpha$_1$-antitrypsine (alpha$_1$-AT). Elle appartient à une famille d'inhibiteurs de protéases appelée serpines, et elle est synthétisée par les hépatocytes, les macrophages, et les monocytes. L'alpha$_1$-antitrypsine est un inhibiteur des sérine-protéases, qui représentent le plus grand groupe des enzymes chez les mammifères. Elle est un inhibiteur majeur des enzymes suivantes: élastase, catepsine G, collagénase, trypsine, chymotrypsine, facteur XIa, urokinase. On pense qu'une déficience en alpha$_1$-AT, associée à des maladies telles que la polyarthrite rhumatoide, l'uvréite antérieure, le lupus erythémateux et d'autres encore, suggère que l'alpha$_1$-AT pourrait être importante non seulement en tant que protéine anti-inflammatoire mais aussi en tant qu'immunomodulateur.

Selon la demande de brevet européen publiée EP-A2-67 293, on utilise le plasma sanguin humain comme matière de départ pour préparer l'alpha$_1$-AT. On réduit chimiquement les liaisons disulfure de protéines contaminantes, et l'on obtient après tout un nombre d'étapes successives, une purification de l'anti-enzyme par un facteur de 15,3 avec un rendement de 50%.

Une utilisation plus étendue des inhibiteurs de protéases est désirable car on a constaté des effets bénéfiques de cette anti-enzyme en pharmacie et en cosmétique. Cette utilisation est à présent limitée car on doit disposer du sérum humain, matière de départ difficile à obtenir et rare, pour obtenir cet inhibiteur.

Le but de la présente invention est donc de trouver une fraction inhibitrice de protéases, comprenant plusieurs facteurs inhibiteurs, pouvant être obtenue à un prix plus modique. Un autre but de l'invention est de mettre à disposition une composition inhibitrice ayant une activité plus universelle et plus grande en soi, étant d'une pureté acceptable pour une utilisation en cosmétique.

De façon tout à fait inattendue, on a trouvé que, si l'on dérive la fraction non pas du sérum humain mais du sérum sanguin chevalin, non seulement elle peut être obtenue à frais réduits, mais elle contient en plus un nouveau facteur, donc inconnu jusqu'à ce jour, et finalement elle a une activité plus universelle et plus étendue.

La fraction purifiée d'inhibiteurs de protéases est définie dans la première revendication indépendante. Le nouveau facteur (horse inhibitor) de la fraction fait l'objet d'une seconde revendication indépendante tandis que les préparations cosmétiques sont définies dans d'autres revendications.

On obtient la nouvelle fraction de préférence par un procédé nouveau et original qui fait l'objet de la demande de brevet suisse no. 3760/89 du 16 octobre 1989.

La première étape de ce procédé comprend la purification du sérum chevalin qui est utilisé comme produit de départ. On peut utiliser le sérum juste après son obtention. Il est également possible d'utiliser le sérum reconstitué obtenu par dissolution du sérum lyophilisé dans une solution saline physiologique, ou de préférence dans une solution tampon, par exemple un tampon au phosphate. Cette solution est de préférence dialysée pendant un certain temps pour enlever des sels indésirables.

La prochaine étape comprend la séparation de l'albumine de la solution tamponnée éventuellement dialysée. Il est à noter que, contrairement aux procédés connus où la plupart des protéines sont précipitées, entraînant nécessairement une certaine perte d'antiprotéases, le procédé utilisé se contente d'enlever l'albumine. On utilise pour cette séparation de préférence un procédé de chromatographie d'affinité en colonne comportant un gel adsorptif. Il s'agit en l'espèce de l'agarose coloré par du Coomassie, un colorant bleu réactif à anneau triazine. Cet agarose retient l'albumine à 100% mais laisse passer les autres protéines. La colonne peut facilement être régénérée avec une solution de chlorure de sodium.

L'étape suivante est la séparation des protéines restantes par concentration isoélectrique ("isoelectrofocusing"). Cette méthode est basée sur le phénomène que les protéines, sous l'influence d'un champ électrique superposé à un gradient de pH, migrent dans la zone où le pH est égal au pI (point isoélectrique) de protéine.

Pour mettre en pratique cette méthode, il existe un appareil appelé "Rotofor", commercialisé par la société Bio-Rad, Richmond, CA (USA). Cet appareil permet la concentration de plus de 25 fois, avec séparation simultanée, de chaque protéine d'un mélange après 4 heures seulement, et il comprend 20 chambres pour 20 fractions.

Ce qui importe, c'est la méthode de concentration isoélectrique préparative et non pas l'utilisation d'un appareil particulier, bien que le Rotofor donne des résultats appropriés.

Avant de soumettre la solution de protéines exempte d'albumine à la concentration isolélectrique préparative, on lui fait subir une dialyse rapide contre l'eau distillée ou déminéralisée, ou bien une chromatographie, afin d'enlever la plupart des sels du tampon. Un liquide à concentrer isoélectriquement ne doit pas présenter une conductivité trop élevée afin qu'un champ électrique de quelques 20 à 80 V/cm puisse s'établir. La solution est séparée en plusieurs échantillons, chacun est dilué avec un ampholyte ayant un pH différent, et l'échantillon est introduit dans l'appareil, chacun dans une cellule séparée, en observant un gradient unidirectionnel du pH. Après concentration isoélectrique, les cellules sont vidées, chacune dans un récipient séparé.

Ensuite, les fractions acides ayant un pH compris entre 3 et 5 environ, plus spécialement entre 3,5 et 4,0, sont travaillées pour isoler le mélange d'antiprotéases intéressé. Les inhibiteurs peuvent alors être séparés des ampholytes solubilisants par des méthodes connues en soi.

Le procédé prévoit aussi la séparation par chromatographie d'affinité en colonne sur un agarose comportant une protéase liée, par exemple l'élastase. Lors de la chromatographie, les inhibiteurs se fixent sur les protéases immobilisées, et ils peuvent être récupérés par élution en utilisant une solution ayant un autre potentiel ionique - changement du pI - et/ou une molarité différente de sels.

On peut finalement séparer le mélange d'inhibiteurs obtenu selon le poids moléculaire, pour obtenir les facteurs du mélange séparément. Une des méthodes de séparation est alors la chromatographie liquide à moyenne ou haute performance en phase renversée (MPLC ou HPLC, respectivement).

Le procédé décrit permet la préparation à partir d'un sérum chevalin d'un mélange d'inhibiteurs de protéase contenant cinq inhibiteurs individuels au moins, à savoir l'alpha$_1$-antichymotrypsine, l'alpha$_1$-antitrypsine, l'antithrombine III, le dimère de l'antitrypsine/antichymotrypsine, et un nouvel inhibiteur de protéases, inconnu jusqu'à ce jour, mais aussi de chaque inhibiteur de protéases séparé. Le mélange contient encore une alpha$_1$-glycoprotéine, du fibrinogène, et d'autres protéines en quantités négligeables. L'activité du mélange et des différents facteurs à été déterminée par l'action sur l'élastase.

On a isolé le nouvel inhibiteur ou facteur lorsqu'on a fractionné le mélange d'inhibiteurs finalement obtenu. Ce nouvel inhibiteur exerce une action inhibitrice sur l'élastase et présente un poids moléculaire d'environ 38 kD (kilodalton) et un point isoélectrique d'environ 4,2. Cet inhibiteur est absent dans le sérum humain. Il est appelé provisoirement "horse inhibitor" d'après sa provenance.

L'invention sera expliquée plus en détail par des exemples qui ne sont qu'illustratifs et ne limitent pas l'invention.

Exemple 1

1. - Préparation de la matière première

Du sérum chevalin lyophilisé est solubilisé, à raison de 1,5 g dans 5 ml de liquide, dans un tampon au phosphate 50 mM, pH 8 (PBS).

2. - Chromatographie d'affinité

Une colonne contenant de l'agarose bleu (colorant réactif triaziné "Coomassie" ou "CIBACRON Blue F3GA" lié à l'agarose) est équilibrée avec 50 ml de PBS.

Le sérum reconstitué et tamponné est chromatographié sur la colonne ce qui a pour résultat l'enlèvement à pratiquement 100% de l'albumine sans altération des autres protéines.

La colonne peut être régénérée par une solution 2M de NaCl et rinçage extensif avec PBS 50 mM.

3. - Concentration isoélectrique sur Rotofor

a. On effectue une dialyse rapide au cours de 3 à 4 heures contre l'eau distillée des effluents de la colonne d'affinité. On dilue ensuite les effluents dialysés réunis à un volume de 50 ml avec des solutions aqueuses d'ampholytes concentrés de 40%, diluées à 2% en poids.

b. Ces solutions aqueuses contiennent alors chacune 2% en poids d'un ampholyte dans la gamme des points isoélectriques de 3 à 10. Il a été trouvé avec surprise que la concentration normale et recommandée de 1% d'ampholyte ne donne pas satisfaction, et qu'une augmentation de cette concentration à 2% a fourni une séparation satisfaisante. De même, les concentrations isoélectriques de protéines semblables se font normalement avec les ampholytes d'un pI de 5 à 7, et non de 3 à 10.

c. L'étape de la concentration isoélectrique à été conduite pendant 4 heures avec une puissance de 12 W. L'appareil est arrêté lorsqu'un plafond de voltage de 1000 à 1200 V est atteint. Au cours du travail, le contenu de toutes les cellules du Rotofor est maintenu à une température de 3ºC environ.

d. L'appareil est vidé, chaque cellule dans un récipient séparé, et les protéines sont séparées de l'ampholyte par une dialyse poussée contre une solution 2M de NaCl; ensuite contre 1M PBS, et les protéines ayant un pI de 3,5 à 4,0 ont été récupérées.

4. - Séparation des protéines à partir des ampholytes

On a préparé une colonne d'agarose dans laquelle on a lié de l'élastase ou une autre protéase à l'agarose. Les fractions acides obtenues dans l'étape de concentration isoélectrique dans lesquelles une activité d'antiprotéase avait été trouvée, ont été filtrées séparément par cette colonne. Les antienzymes ont été réversiblement retenues, et on les a récupérées en éluant avec un tampon de pouvoir ou concentration ioniques différent.

Le contenu de chaque fraction a été finalement contrôlé pour l'activité antiprotéase par une électrophorèse bidimensionnelle.

Une activité pour inhiber l'élastase a été trouvée pour les fractions 1 à 5 parmi les 20 fractions sortant du Rotofor, correspondant à un pI compris entre 3,5 et 4,0. Ces cinq fractions ont été réunies et analysées plus en détail. Une activité inhibitrice a aussi été constatée concernant la trypsine, la chymotrypsine, la plasmine et la collagénase.

La fraction acide combinée peut être séparée en protéines individuels par électrophorèse sur gel de polyacrylamide uni et bidimensionnelle. On a trouvé 5 protéines majeures ayant des poids moléculaires compris entre 46 et 68 kD, plus un sixième facteur, poids moléculaire 38 kD, pI 4,2, actif contre l'élastase, qu'on a appelé "horse inhibitor". Le degré de purification est supérieur aux préparations d'antienzymes connues.

Une analyse type de la nouvelle composition inhibitrice de protéases est la suivante:

## Tableau I

| Facteur no. | poids mol. (kD) | pI (A) | inhibition sur l'élastase | nature |
|---|---|---|---|---|
| 1 | 58 | 4,3 | ± | $\alpha$ 1-$\beta$-glyco-protéine |
| 2 | 55 | 3,9 | + | $\alpha$ 1-antichymo-trypsine |
| 3 | 53 | 4,0 | + | $\alpha$ 1-antitrypsine + antithrombine III (traces) |
| 4 | 51 | 4,5 | − | fibrinogène (chaîne $\gamma$) |
| 5 | 50 | 4,1 | − | $\alpha$ 2-glyco-protéine |
| 6 | 38 | 4,2 | ++ | "facteur cheval" (nouveau) |

Exemples 2 - 4

Préparations cosmétiques

Les exemples qui suivent décrivent trois préparations cosmétiques. Les ingrédients sont désignés normalement par leurs noms en langue anglaise, recommandés et couramment utilisés selon le CTFA Cosmetic Ingredient Dictionary, The Cosmetic, Toiletry and Fragrance Association, Inc., Washington, D.C., USA.

Exemple 2

Crème nourissante

A. Composition

| Groupe | dénomination CTFA | % en poids/poids |
|--------|-------------------|------------------|
| (2) | Eau | 61,33 |
| (1) | PEG-6 stearate (et) Glyceryl stearate (et) CETETH 20 | 15,00 |
| (5) | Hydrolyzed elastin | 6,00 |
| (1) | Mineral oil | 5,00 |
| (1) | Wheat germ oil | 3,00 |
| (1) | Sweet almond oil | 2,00 |
| (1) | Isosteryl isostearate | 2,00 |
| (1) | Cetyl alcohol | 1,50 |
| (4) | PFPI (fraction purifiée d'inhibiteurs de protéase) | 1,00 |
| (1) | Octyl dodecyl myristate | 1,00 |
| (1) | Lanolin wax | 1,00 |
| (6) | Fragrance (PCV 1468 - Givaudan) | 0,30 |
| (2) | Imidazolidinyl urea | 0,30 |
| (3) | Triethanolamine | 0,22 |
| (2) | Carbomer 934 | 0,20 |
| (2) | Methylchloroisothiazolinone (et) Methylisothiazolinone | 0,10 |
| (1) | BHA (et) BHT (et) Propyl gallate (et) Citric acid | 0,05 |
| | | ------ |
| | | 100,00 |

B. Mode de préparation

Disperser le Carbomer 934 à l'aide d'un homogénéisateur et laisser reposer une nuit. Chauffer les constituants du groupe (1) et (2) à 75°C et verser (2) dans (1) à 75°C. Refroidir sous agitation. A 35°C, ajouter les autres constituants dans l'ordre des numéros. Terminer le refroidissement sous agitation.

Exemple 3

Lait corporel hydratant

A. Composition

| Groupe | dénomination CTFA | % en poids/poids |
|---|---|---|
| (1) | Eau | 78,500 |
| (2) | Mineral oil | 8,500 |
| (2) | Glycerine | 3,000 |
| (2) | Stearic acid | 2,750 |
| (2) | Caprylic/capric triglyceride | 1,500 |
| (2) | Octyldodecanol | 1,500 |
| (2) | Triethanolamine | 1,000 |
| (2) | Acetylated lanolin | 0,800 |
| (2) | Jojoba oil | 0,500 |
| (2) | Cetyl alcohol | 0,500 |
| (4) | PFPI (fraction purifiée d'inhibiteurs de protéase) | 0,500 |
| (6) | Fragrance (PCV 1563) | 0,400 |
| (2) | Phenoxyethanol (et) Methyl (et) Ethyl (et) Butyl (et) Propyl paraben | 0,200 |
| (3) | Sodium dehydroacetate | 0,200 |
| (1) | Carbomer 941 | 0,100 |
| (6) | Tricolosan | 0,050 |

B. Mode de préparation

Disperser le Carbomer 941 (1) dans l'eau (1). Laisser reposer une nuit. Chauffer à 75°C les constituants (2), puis verser (2) dans (1). Refroidir sous agitation jusqu'à 35°C. Puis ajouter les autres constituants dans l'ordre des numéros. Terminer le refroidissement sous agitation.

Exemple 4

Gel facial

A. Composition

| Groupe | dénomination CTFA | % en poids/poids |
|--------|-------------------|------------------|
| (1) | Eau | 79,8747 |
| (9) | Soluble animal collagen (et) mixed mucopolysaccharides | 10,0000 |
| (5) | Dimethicone copolyol | 5,0000 |
| (4) | Glycerine | 2,0000 |
| (8) | Propylene glycol | 1,0000 |
| (4) | PFPI (fraction purifiée d'inhibiteurs de protéase) | 0,5000 |
| (16) | Polyquaternium – 11 | 0,5000 |
| (3) | Triethanolamine | 0,3750 |
| (11) | Octoxynol 11 (et) Polysorbate 20 | 0,2250 |
| (1) | Carbomer 941 | 0,2000 |
| (2) | Methylchloroisothiazolinone (et) Methylisothiazolinone | 0,1500 |
| (7) | Hyaluronic acid | 0,1000 |
| (10) | Perfume | 0,0750 |
| (13) | Red colouring | 0,0003 |

B. Mode de préparation

Disperser le Carbomer 941 (1) dans l'eau (1). Laisser reposer une nuit. Ajouter les autres constituants dans l'ordre de la formule sous agitation. Si la fabrication n'est pas faite sous vide, dégazer par centrifugation.

Le facteur "horse inhibitor" ainsi que la fraction inhibitrice le contenant trouvent une application en médecine, en pharmacie et en cosmétique.

Il va de soi que de nombreuses autres préparations cosmétiques peuvent être élaborées selon les connaissances de l'homme du métier, et l'invention n'est donc pas limitée aux exemples spécifiques donnés ci-dessus.

**Revendications**

1. Fraction purifiée d'inhibiteurs de protéases, comprenant des facteurs exerçant une action inhibitrice de l'élastase au moins, caractérisée en ce qu'elle contient un facteur ayant un poids moléculaire d'environ 38 kD et un point iso-électrique d'environ 4,2, la fraction ayant été obtenue à partir du sérum sanguin chevalin.

2. Fraction selon la revendication 1, caractérisée en ce qu'elle contient en plus un facteur inhibiteur de la chymotrypsine.

3. Fraction selon la revendication 1, caractérisée en ce qu'elle contient en plus un facteur inhibiteur de la trypsine.

4. Fraction selon la revendication 1, caractérisée en ce qu'elle contient en plus un facteur inhibiteur de la thrombine-III.

5. Fraction selon la revendication 1, caractérisée en ce qu'elle contient en plus une $\alpha$ 1-$\beta$-glycoprotéine.

6. Fraction selon la revendication 1, caractérisée en ce qu'elle contient en plus une $\alpha$ 2-glycoprotéine.

7. Fraction selon la revendication 1, caractérisée en ce qu'elle contient en plus du fibrinogène.

8. Facteur inhibiteur d'élastase, obtenu à partir du sérum sanguin chevalin, ayant un poids moléculaire d'environ 38 kD et un point iso-électrique d'environ 4,2.

9. Préparation cosmétique, contenant comme ingrédient actif la fraction selon l'une quelconque des revendications 1 à 7.

10. Préparation cosmétique selon la revendication 9, caractérisée en ce qu'elle contient le facteur selon la revendication 8.

11. Préparation cosmétique selon l'une des revendications 5, 9 et 10, caractérisée en ce qu'elle est une crème nourrissante.

12. Préparation cosmétique selon l'une des revendications 5, 9 et 10, caractérisée en ce qu'elle est un lait corporel.

13. Préparation cosmétique selon l'une des revendications 5, 9 et 10, caractérisée en ce qu'elle est un gel facial.

8

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | BIOCHIMICA ET BIOPHYSICA, vol. 797, 1984, pages 336-342, Elsevier Science Publishers B.V.; A. PELLEGRINI et al.: "The isolation and characterization of a new elastase inhibitor, pre-alpha2-elastase inhibitor, of the horse" * En entier * --- | 1-8 | C 07 K 15/06 A 61 K 7/00 |
| A | CHEMICAL ABSTRACTS, vol. 90, no. 17, 23 avril 1979, page 325, résumé no. 135633n, Columbus, Ohio, US; R. VON FELLENBERG: "Electrophoretic analysis of protease inhibitors in horses serum", & SCHWEIZ. ARCH. TIERHEILKD. 1978, 120(12), 631-42 * Résumé * --- | 1-8 | |
| A | US-A-3 912 704 (KARTAR SINGH) * Résumé * ----- | 1-8 | |

DOMAINES TECHNIQUES
RECHERCHES (Int. Cl.5)

A 61 K
C 07 K

Le présent rapport a été établi pour toutes les revendications

| Lieu de la recherche | Date d'achèvement de la recherche | Examinateur |
|---|---|---|
| LA HAYE | 19-02-1991 | FERNANDEZ Y BRANAS F.J. |